# FASCICULE DE BREVET EUROPEEN

(11) **EP 2 208 781 B1**
(45) Date de publication et mention de la délivrance du brevet: **22.08.2012**
(21) Numéro de dépôt: 10156301.3
(22) Date de dépôt: 21.04.2006
(51) Int. Cl.: C12N 1/36, C12P 19/04, C12N 1/20

(54) **Procédé de production de souches de Staphylococcus aureus surproductrices**
Verfahren zur Herstellung von Stämmen vonüberproduktivem Staphylococcus aureus
Methods for producing strains of overproductive staphylococcus aureus

(30) Priorité: 25.04.2005 FR 0504115
(43) Date de publication de la demande: 21.07.2010
(62) Demande divisionnaire de: 06755433.7
(73) Titulaire: Sanofi Pasteur, 69367 Lyon Cedex 07 (FR)
(72) Inventeur: ROKBI, Bachra, 69003 Lyon (FR); LAFONT CIVAT, Céline, 42140 Marcenod (FR)

(56) Documents cités:
- POUTREL B, ET AL.: "EFFECTS OF CULTURE CONDITIONS ON PRODUCTION OF TYPE 5 CAPSULAR POLYSACCHARIDE BY HUMAN AND BOVINE STRAPHYLOCOCCUS AUREUS STRAINS" CLINICAL AND DIAGNOSTIC LABORATORY IMMUNOLOGY, vol. 2, no. 2, 1995, pages 166-171, XP009051201 & TAYLOR D AND HOLLAND K T: "AMINO ACID REQUIREMENTS FOR THE GROWTH AND PRODUCTION OF SOME EXOCELLULAR PRODUCTS OF STRAPHYLOCOCCUS AUREUS" JOURNAL OF APPLIED BACTERIOLOGY, vol. 66, 1989, pages 319-329, XP009051284
- STRINGFELLOW W T, ET AL.: "STAPHYLOCOCCUS AUREUS GROWTH AND TYPE 5 CAPSULAR POLYSACCHARIDE PRODUCTION IN SYNTHETIC MEDIA" APPLIED AND ENVIRONMENTAL MICROBIOLOGY, vol. 57, no. 2, 1991, pages 618-621, XP009051269

## Description

La présente invention concerne un milieu de pré-culture adapté à la culture de *Staphylococcus aureus.*

Les polysaccharides capsulaires de *Staphylococcus aureus,* en particulier les polysaccharides de sérotypes 5 et 8, sont décrits dans la littérature comme des composants d'intérêt pour la fabrication d'un vaccin visant à protéger contre les infections dues à *Staphylococcus aureus.*

Les souches T5 et T8 sont microcapsulées et produisent donc de faibles quantités de polysaccharides capsulaires d'intérêt. Les quantités de polysaccharides capsulaires devant être inclues dans un vaccin sont importantes du fait du système immunitaire déficient des populations ciblées (voir par exemple Fattom, A., et al (1995) Vaccine 13, 1288-1293).

Dans le cadre d'un développement industriel d'un tel vaccin il est donc important de pouvoir produire de grandes quantités de polysaccharides capsulaires T5 et T8.

C'est dans cette optique que les inventeurs ont mis en évidence un nouveau procédé permettant d'augmenter substantiellement la capacité de sécrétion de polysaccharide capsulaire des souches de *Staphylococcus aureus.* Les inventeurs fournissent donc un procédé de production permettant d'obtenir du polysaccharide capsulaire de *Staphylococcus aureus* en grandes quantités, un procédé de production de souches de *Staphylococcus aureus* surproductrices comprenant :
(a) la culture d'une souche de *Staphylococcus aureus* sur un milieu de culture M1 comprenant par litre de milieu de culture :
   - 5 à 100 g de peptone végétale,
   - 0,5 à 20 g d'extrait de levure,
   - 1 à 30 g de sucre,
   - 1 à 200 mg d'un sel de fer sélectionné dans le groupe consistant en FeCl₃ et FeSO₄,
   - 1 à 700 mg d'un sel de magnésium sélectionné dans le groupe consistant en MgCl₂ et Mg SO₄,
   - 0,1 à 500 mg de CaCl₂,
   - 0 à 50 mg de ZnCl₂,
   - 10 à 250 g de NaCl, de préférence 58,5 g/l, et
   dont le pH est compris dans une gamme de valeurs allant de 6,2 à 7,5, et
(b) éventuellement la récupération des souches à partir du milieu de culture.

Selon un mode de réalisation particulier du procédé de production de souches surproductrices, le milieu de culture M1 de l'étape (a) comprend de la peptone de blé.

Selon un mode de réalisation avantageux du procédé de production de souches surproductrices, le milieu de culture M1 comprend par litre de milieu de culture:
- 30 g de peptone de blé,
- 5 g d'extrait de levure,
- 1 g de D-glucose H₂O,
- 120 mg de FeCl₃,6H₂O,
- 400 mg de MgCl₂,6H₂O,
- 10 mg de CaCl₂,2H₂O,
- 58,5 g de NaCl,
- 5 mg de ZnCl₂ ,
- 6.8 g de (NH₄)₂SO₄,
- 6 g de NH₄Cl et pH =6,5
Selon un autre mode de réalisation avantageux le milieu M1 consiste par litre en : 30 g de peptone de blé, 5 g d'extrait de levure, 1 g de D-glucose H₂O,120 mg de FeCl₃,6H₂O, 400 mg de MgCl₂,6H₂O, 10 mg de CaCl₂,2H₂O, 58,5 g de NaCl, 5 mg de ZnCl₂, 6.8 g de (NH₄)₂SO₄ et 6 g de NH4Cl, pH=6,5.

Selon un mode de réalisation particulier le procédé de production de souches surproductrices comprend une étape de pré-culture sur un milieu de pré-culture M0 comprenant :
- 3 à 50 g de peptone végétale,
- 0,5 à 20 g d'extrait de levure,
- 1 à 30 g de sucre,
- 1 à 200 mg d'un sel de fer sélectionné dans le groupe consistant en FeCl₃ et FeSO₄,
- 1 à 700 mg de sel de magnésium sélectionné dans le groupe consistant en MgCl₂ et Mg SO₄,
- 0,1 à 50 mg de CaCl₂,
- 0 à 50 mg de ZnCl₂,
- 2 à 50 g de NaCl, de préférence 6 g/l, et
dont le pH est compris dans une gamme de valeurs allant de 6,2 à 7,5.

Selon un mode particulier de réalisation du procédé de production de souches surproductrices, l'étape de pré-culture utilise un milieu de pré-culture M0 comprenant par litre 3,87 g de peptone de blé.

Selon un autre mode de réalisation du procédé de production de souches surproductrices, l'étape de pré-culture utilise un milieu de pré-culture M0 comprenant par litre 30 g de peptone de blé.

Selon un mode de réalisation avantageux du procédé de production de souches surproductrices, l'étape de pré-culture utilise un milieu de pré-culture M0 gélosé comprenant: 3,87 g de peptone de blé, 5 g d'extrait de levure, 1 g de D-glucose H₂O,120 mg de FeCl₃,6H₂O, 400 mg de MgCl₂,6H₂O, 10 mg de CaCl₂,2H₂O, 6 g de NaCl, 5 mg de ZnCl₂, 6.8 g de (NH₄)₂SO₄, 6 g de NH₄Cl, 15g/l d'agar et pH = 6,5.

Selon un autre mode de réalisation avantageux du procédé de production de souches surproductrices, l'étape de pré-culture utilise un milieu de pré-culture M0 liquide comprenant : 30 g de peptone de blé, 5 g d'extrait de levure, 1 g de D-glucose H₂O,120 mg de FeCl₃,6H₂O, 400 mg de MgCl₂,6H₂O, 10 mg de CaCl₂,2H₂O, 6 g de NaCl, 5 mg de ZnCl₂, 6.8 g de (NH₄)₂SO₄, 6 g de NH₄Cl, et pH= 6,5.

Selon un mode de réalisation le procédé tel que défini ci-dessus est un procédé de production de souches surproductrices de *Staphylococcus aureus* T5. Selon un autre mode de réalisation le procédé tel que défini ci-dessus est un procédé de production de souches surproductrices de *Staphylococcus aureus* T8.

Ce procédé de production de polysaccharides capsulaires comprend :
(a) la culture d'une souche de *Staphylococcus aureus* sur un milieu de culture M1 tel que défini ci-dessus,
(b) l'inactivation de la culture ainsi produite, et
(c) la récupération des polysaccharides capsulaires.

Selon un mode de réalisation du procédé de production de polysaccharides capsulaires l'étape de culture utilise un milieu de culture M1 comprenant de la peptone de blé.

Selon un mode de réalisation le procédé de production de polysaccharide capsulaire comprend une étape de pré-culture telle que définie ci-dessus en relation avec le procédé de production de souches surproductrices.

La présente invention a pour objet un milieu de pré-culture M0 adapté à la culture de *Staphylococcus aureus* comprenant :
- 3 à 50 g de peptone végétale,
- 0,5 à 20 g d'extrait de levure,
- 1 à 30 g de sucre,
- 1 à 200 mg d'un sel de fer sélectionné dans le groupe consistant en FeCl₃ et FeSO₄,
- 1 à 700 mg de sel de magnésium sélectionné dans le groupe consistant en MgCl₂ et Mg SO₄.
- 0,1 à 50 mg de CaCl₂,
- 0 à 50 mg de ZnCl₂,
- 2 à 50 g de NaCl, de préférence 6 g/l, et
dont le pH est compris dans une gamme de valeurs allant de 6,2 à 7,5, et concerne de préférence un milieu M0 gélosé ou liquide comprenant par litre et avantageusement consistant en : 3,87g ou 30 g de peptone de blé et 5 g d'extrait de levure, 1 g de D-glucose H₂O,120 mg de FeCl₃,6H₂O, 400 mg de MgCl₂,6H₂O, 10 mg de CaCl₂,2H₂O, 6 g de N_{A}Cl, 5 mg de ZnCl₂ , 6.8 g de (NH₄)₂SO₄, 6 g de NH₄Cl et pH = 6,5.

Les inventeurs ont mis en évidence que le milieu M1 tel que défini ci-dessus pouvait être avantageusement remplacé par un milieu simplifié M2 dans le procédé de production de souches de *S. aureus* surproductrices et le procédé de production de polysaccharides capsulaires tels que définis ci-dessus.
Ce milieu de culture M2 comprend par litre de milieu de culture :
- 52,5 à 100 g de peptone végétale, avantageusement de la peptone de blé,
- 0 à 82,5 g de NaCl, avantageusement de 5g à 82,5g, en particulier 41g/l de NaCl
- 0 à 15g/l d'un sel de magnésium, avantageusement de 0,01 à 15g/l, en particulier 15g/l de MgCl₂,6H₂O et
- 0 à 7g/l de sucre, avantageusement de 0,25 à 7g/l, en particulier 0.25g/l de D-glucose H₂O,
dont le pH est compris dans une gamme de valeurs de 6.3 à 6.7 avantageusement de 6.4 à 6.6.
Selon un mode de réalisation particulier le milieu de culture M2 comprend, et avantageusement consiste en, 93g/l de peptone de blé, 41g/l de NaCl, 0,25g/l de D-glucose H₂O et 15g/l de MgCl₂,6H₂O dont le pH est dans une gamme de valeurs de 6.3 à 6.7.

Les inventeurs ont également mis en évidence qu'un milieu de culture M3, correspondant à un milieu M2 dans lequel la peptone végétale est remplacée par 65 à 85g /l, avantageusement 75g/l d'extrait de levure pouvait être utilisé dans le procédé de production de souches de S. aureus surproductrices et le procédé de production de polysaccharides capsulaires tels que définis ci-dessus.
Ce milieu de culture M3 comprend par litre de milieu de culture :
- 65 à 85 g d'extrait de levure, avantageusement 75g/l d'extrait de levure,
- 0 à 82,5 g de NaCl, avantageusement de 5g à 82,5g, en particulier 41g/l de NaCl
- 0 à 15g/l d'un sel de magnésium, avantageusement de 0,01 à 15g/l, en particulier 15g/l de MgCl₂,6H₂O et
- 0 à 7g/l de sucre, avantageusement de 0,25 à 7g/l, en particulier 0.25g/l de D-glucose, H₂O,
dont le pH est compris dans une gamme de valeurs allant de 6.3 à 6.7.

Les inventeurs ont mis en évidence de façon surprenante que les milieux de culture M1, M2 et M3 induisent une augmentation substantielle de la capacité de sécrétion de polysaccharide capsulaire des souches de *Staphylococcus aureus.* Ladite augmentation peut être mise en évidence par mesure de la quantité de polysaccharide capsulaire produite par la méthode ELISA telle que décrite dans l'exemple 2.4 ci-dessous. Lesdites souches de *Staphylococcus aureus* présentant une capacité de sécrétion accrue sont nommées dans le cadre de la présente invention souches surproductrices. On entend donc dans le cadre de la présente invention par «souches surproductrices » une souche de *Staphylococcus aureus* qui après culture sur un milieu M1 ou M2 ou M3 produit une quantité de polysaccharide capsulaire en mg/l, telle que déterminée par la méthode ELISA décrite dans l'exemple 2.4, au moins 3 fois, de préférence 5 fois et avantageusement 10 fois supérieure, à celle produite par culture de la même souche de départ sur le milieu de Poutrel (voir Poutrel, B et al. (1995). Clin Diagn Lab Immunol 2, 166-171 pour un descriptif du milieu ou l'exemple 1.1 ci-dessous)) dans les mêmes conditions de culture.

Dans le cas d'une souche recombinante, on entend par « souches surproductrices » une souche de *Staphylococcus aureus* recombinante qui après culture sur un milieu de culture M1 ou M2 ou M3 produit une quantité de polysaccharide capsulaire en mg/l, telle que déterminée par la méthode ELISA décrite dans l'exemple 2.4, au moins 2 fois supérieure, à celle produite par culture de la même souche de départ sur le milieu TSB (voir exemple 1.5 pour un descriptif du milieu) dans les mêmes conditions de culture.

Les inventeurs ont mis en évidence de façon surprenante que le procédé de production de souches surproductrices était applicable aux souches de *Staphylococcus aureus* T5 et aux souches T8. Les inventeurs ont également observé que le procédé selon l'invention était également applicable aux souches recombinantes qui ont été modifiées génétiquement pour surproduire leur polysaccharide capsulaire. Les inventeurs ont démontré cet aspect en appliquant le procédé selon l'invention à la souche CYL770 génétiquement modifiée pour produire du polysaccharide capsulaire T8 en grandes quantités. La souche après culture sur milieu M1 ou M2 ou M3 voit sa capacité de sécrétion en polysaccharide capsulaire T8 augmenter d'un facteur 2 comme cela est décrit dans les exemples ci-dessous.

On entend donc dans le cadre de la présente invention par «souches de *Staphylococcus aureus*» toute souche de *Staphylococcus aureus* exprimant une capsule ou microcapsule comprenant un polysaccharide de sérotype T5 et/ou T8. On peut citer à titre d'exemple non limitatifs de souches appropriées les souches Newman (T5), Reynolds(T5), Lowenstein (T5), Becker(T8), Wright (T8) et CYL770 (T8) et CYL1892 (T5).
Ces souches sont disponibles auprès de divers instituts ou dans diverses collections, on peut en particulier citer la collection de l'Institut Pasteur (CIP) : Reynolds CIP 103313, Becker CIP103314, ou l'ATCC : Wright ATCC9525, Loweinstein ATCC 49521. Dans le cadre de la présente invention on utilise de préférence les souches CYL770 (T8) et Reynolds (T5). La souche CYL770 est une souche Becker modifiée génétiquement pour produire de plus grandes quantités de polysaccharide capsulaire T8. Cette souche peut être avantageusement utilisée comme souche de départ dans le procédé de production de souches surproductrices selon l'invention ainsi que dans le procédé de production de polysaccharide selon l'invention. Un descriptif de la souche CYL770 est donné dans l'article de Luong, T.T et Lee, C.Y (2002), Infect.Immun. 70: 3389-3395. La souche CYL1892 désigne la souche de *S. aureus* recombinante, obtenue à partir d'une souche Reynolds (CIP 103313) dans laquelle le promoteur de l'opéron *cap5* a été remplacé par le promoteur constitutif fort de l'opéron cap1 de la souche M de *S. aureus*, selon le procédé décrit par Luong et Lee (2002 Infect.Immun. 70: 3389-3395) avec les modifications suivantes : les séquences homologues ont été remplacées par des séquences équivalentes homologues de la souche Reynolds, le plasmide pCL52.2 a été remplacé par le plasmide pCL10 contenant une origine de réplication thremo-sensible et comprenant un marqueur cat (chloramphenicol acetyl tranferase conférant une résistance au chloramphénicol). Brièvement un fragment d'ADN d'environ 1 kb contenant l'ORF *cap5* de la souche Reynolds fusionné à un fragment d'environ 250-bp contenant le P*cap1* promoteur est construit par la technique de PCR par chevauchement. Un fragment d'ADN d'environ 1 kb contenant la séquence amont du gène *cap5A* est amplifié par PCR à partir du génome de la souche Reynolds. Les deux fragments sont ensuite clonés dans le plasmide pCL10 portant une origine de réplication sensible à la température et comportant un marqueur cat de sorte que le promoteur P*cap5* soit remplacé par le promoteur P*cap1*. Le plasmide ainsi construit est ensuite électroporé dans une souche RN4220 puis introduit par transduction à l'aide du bactériophage 52 dans la souche Reynolds. La sensibilité de l'origine de réplication à la température du plasmide permet ensuite de favoriser la recombinaison homologue en bloquant sa réplication tout en appliquant une sélection par le chloramphénicol.

On entend par « polysaccharide capsulaire » dans le cadre de la présente invention les polysaccharides capsulaires T5 et/ou T8 des souches de *Staphylococcus aureus* ainsi que les fragments en dérivant.

On entend par « milieu de culture » dans le cadre de la présente invention un milieu exempt de protéine d'origine animale.

Par « protéine d'origine animale » on entend les protéines obtenues à partir de matière d'origine animale ainsi que tout produit en dérivant, tels que les dérivés issus du traitement chimique des protéines animales. On entend également les produits de l'hydrolyse partielle ou totale de ces protéines animales tels que les peptones, les polypeptides, les peptides, ou les acides aminés en dérivant.

Le procédé de production d'une souche surproductrice selon l'invention comprend (a) une étape de culture d'une souche de *Staphylococcus aureus*. Selon un mode de réalisation, l'étape de culture utilise un milieu de culture M1 comprenant :
- 5 à 100 g de peptone végétale,
- 0,5 à 20 g d'extrait de levure,
- 1 à 30 g de sucre,
- 1 à 200 mg d'un sel de fer sélectionné dans le groupe consistant en FeCl₃ et FeSO₄,
- 1 à 700 mg d'un sel de magnésium sélectionné dans le groupe consistant en MgCl₂ et Mg SO₄,
- 0,1 à 50 mg de CaCl2,
- 0 à 50 mg de ZnCl2,
- 10 à 250 g de NaCl, de préférence 58,5 g/l, et
dont le pH est compris dans une gamme de valeurs allant de 6,2 à 7,5.

La source d'azote protéique dans le milieu M1 utilisé est apportée principalement par une peptone végétale.

On entend par « peptone végétale » dans le cadre de la présente invention les peptones sélectionnées dans le groupe consistant en : peptone de blé et peptone de pois Les peptones végétales sont généralement sous la forme d'hydrolysats obtenus par traitement enzymatique ou chimique des protéines extraites généralement des parties de la plante qui contiennent les plus fortes teneurs en protéines, telles que le germe de blé par exemple. On utilise de préférence des plantes qui n'ont pas été modifiées génétiquement. Dans le cas des traitements chimiques, l'extrait protéique est soumis à l'action de l'acide chlorhydrique à chaud et sous pression. L'hydrolysat est ensuite neutralisé par de la soude puis débarrassé des sous produits solides. Dans le cas des traitements enzymatiques, l'extrait protéique est digéré habituellement par la papaïne. Les peptones obtenues sont constituées principalement d'un mélange d'acides aminés, et des petits peptides dont le PM est ≤ 1KD. Les peptides dont le PM est > 1 KD représentent moins de 30% du mélange. Les préparations commerciales référencées sous les noms (Hy-Pea 7404 de Quest ref 5710565, Petone de blé El d'Organotechnie ref. 19559) sont des exemples d'hydrolysats de peptones végétales utilisables dans le cadre de la présente invention.
La concentration de peptone végétale utilisée dans le milieu M1 est sélectionnée en tenant compte de la teneur en azote protéique de la peptone et en tenant compte de la concentration en extrait de levure. Cette teneur est calculée par la méthode de Kjeldahl. Pour plus de détails on peut se référer par exemple à l'article de Lynch J.M. et al, J. AOAC Int. (1999) 82 (6): 1389-98. Habituellement les teneurs en azote protéique des peptones végétales sélectionnées sont comprises entre 8% et 15 % ((poids/poids)). Dans cette gamme de valeurs, la concentration en peptone végétale utilisable est de 5 à 100 g par litre de milieu de culture, de préférence de 20 à 50 g/l et en particulier 30g/l.

La source d'azote protéique dans le milieu M1 utilisé est apportée également par de l'extrait de levure. Les teneurs en azote protéique dans les extraits de levure sont généralement de 9 à 13% (poids/poids). L'extrait de levure est utilisé à une concentration de 0,5 à 20 g/l de milieu de culture, et en particulier à une concentration de 5 g/l. L'azote protéique du milieu de culture peut également être apporté sous la forme de sels minéraux, on peut citer à titre exemple les sels d'ammonium, tels que chlorure ou sulfate. Ces sels minéraux peuvent être utilisés à des concentrations qui seront à ajuster en fonction des apports fournis par les autres sources d'azote.

Les concentrations en sources d'azote protéique sont ajustées de telle sorte que la teneur en azote protéique totale ne conduit pas à un hypercatabolisme azoté pouvant être responsable de l'accumulation de déchets toxiques au cours de la culture. D'autre part pour éviter l'apparition d'un effet inhibiteur substantiel d'un excès de vitamines apportées par l'extrait de levure, ce dernier n'est utilisé que comme source secondaire d'azote protéique et de préférence à une concentration de 5 g/l.

Selon un mode de réalisation avantageux, le milieu de culture M1 comprend par litre de milieu 30 g de peptone, de préférence de peptone de blé et 5 g d'extrait de levure.

Le milieu de culture comprend également une source de sucre.

On entend par « source de sucre» dans le cadre de la présente invention, toute source de sucre d'origine non animale, métabolisable par la bactérie, telle que fructose, ribose, xylose, fucose ou glucose. Des concentrations de l'ordre de 1 à 30 g/ litre de milieu de culture peuvent être utilisées. Pour le glucose par exemple une concentration de 1g/l est avantageusement utilisée.

Le milieu de culture M1 comprend également des sels minéraux et en particulier au moins des sels, de préférence des chlorures, de fer, de magnésium, de calcium, de Zinc et de sodium dans les quantités suivantes:
- de 1 à 200 mg d'un sel de fer sélectionné dans le groupe consistant en FeCl₃ et FeSO₄ et en particulier 120 mg de FeCl₃, par litre de milieu de culture ;
- de 1 à 700 mg d'un sel de magnésium sélectionné dans le groupe consistant en MgCl₂ et MgSO₄ et en particulier 400 mg de MgCl₂, par litre de milieu de culture ;
- de 0,5 à 50 mg de CaCl₂ et en particulier 10 mg de CaCl₂, par litre de milieu de culture;
- de 0 à 50 mg de ZnCl₂ et en particulier 5 mg de ZnCl₂ par litre de milieu de culture; et
- de 10 à 250g de NaCl, de préférence 58,5 g par litre de milieu de culture.
Les sels minéraux de Fe, Ca et Mg sont classiquement utilisés sous forme hydratée. Dans le cadre de la présente invention les sels hydratés suivants ont été utilisés dans les milieux de culture : FeCl₃,6H₂O, MgCl₂,6H₂O, CaCl₂,2H₂O.

Le milieu M1 présente un pH initial compris dans une gamme de valeurs allant de 6,2 à 7,5, de préférence un pH de 6,5, pour ce faire tout sel minéral classiquement utilisé pour son pouvoir tampon et permettant d'obtenir un pH dans la gamme de valeur indiquée ci-dessus peut être utilisé si nécessaire. Pour la détermination du pH, la mesure du pH est faite à l'aide d'un pH-mètre à température ambiante.

Le milieu M1 peut également comprendre des agents régulant la pression osmotique tel que glycérophosphate et/ou glycine-bétaïne à des concentrations de 1 à 10 mM pour la glycine bétaine et 50 à 100 mM pour le glycérophosphate, qu'il peut être avantageux d'ajouter lorsque la concentration en NaCl est supérieure à 1 M (58 g/l).

Le milieu de culture M1 utilisé correspond de préférence à un milieu liquide. Il peut cependant se présenter sous forme solide. La forme solide ou gélosée est obtenue par addition au milieu liquide d'une substance gélifiante, habituellement de l'agar à une concentration de 10 à 30 g par litre de milieu de culture.

Selon un mode de réalisation avantageux le milieu de culture M1 comprend, et de préférence consiste en, par litre: 30 g de peptone de blé, 5 g d'extrait de levure, 1 g de D-glucose H₂O,120 mg de FeCl₃,6H₂O, 400 mg de MgCl₂,6H₂O, 10 mg de CaCl₂,2H₂O, 58,5 g de NaCl, 5 mg de ZnCl₂, 6.8 g de (NH₄)₂SO₄ et 6 g de NH₄Cl et présente un pH de 6,5.

L'étape de culture peut être réalisée par ensemencement du milieu M1 avec un inoculum, par exemple avec une DO680 nm initiale de 0,2, et incubation à 37°C pendant une durée par exemple d'environ 48 à 72 heures dans une atmosphère contenant ou pas de CO₂ à une concentration de 5 à 10% (pour le type 5 sans CO₂ et dans le cas de l'utilisation de la souche Becker avec CO₂). Le volume de culture peut varier en fonction des besoins. Des volumes de 400ml à 201 ou des volumes supérieurs par exemple de 301 à 1001 peuvent être utilisés. De préférence un rapport volume du milieu de culture par rapport au volume du récipient de culture de 20 % (V/V) est maintenu. Pour les grands volumes, l'oxygénation est contrôlée et régulée au cours de la culture par un ajustement de la pression en oxygène et une agitation appropriée.
En fonction des besoins, l'étape de culture peut comprendre des cultures successives dans des volumes croissants afin d'augmenter la biomasse et la quantité de polysaccharides produits.

Selon un mode de réalisation avantageux, le procédé de production de souches surproductrices comprend une étape de pré-culture sur un milieu de pré-culture M0 comprenant par litre:
- 1 à 30 g de peptone végétale,
- 0,5 à 20 g d'extrait de levure,
- 1 à 30 g de sucre,
- 1 à 200 mg d'un sel de fer sélectionné dans le groupe consistant en FeCl₃ et FeSO₄,
- 1 à 700 mg d'un sel de magnésium sélectionné dans le groupe consistant en MgCl₂ et Mg SO₄,
- 0,1 à 50 mg de CaCl₂,
- 0,1 à 50 mg de ZnCl₂,
- 0 à 50 g de NaCl, de préférence 6 g/l , et
dont le pH est compris dans une gamme de valeurs allant de 6,2 à 7,5.

Pour les définitions des différents composants on peut se reporter aux définitions données ci-dessus en relation avec le milieu de culture M1.

Par contre, dans le cadre de l'étape de pré-culture, on utilise de préférence 3,87 g ou 30g de peptone, en particulier de peptone de blé par litre de milieu de pré-culture. La concentration en NaCl dans le milieu M0 est de préférence de 6g par litre. Le milieu de pré-culture M0 dans l'étape de pré-culture est de préférence soit un milieu gélosé comprenant 3,87 g de peptone de blé par litre soit un milieu liquide comprenant 30 g de peptone de blé par litre.

Selon un mode de réalisation avantageux, l'étape de pré-culture est mise en oeuvre par culture de la souche sur un milieu pré-culture M0 comprenant, et de préférence consistant en, par litre : 30 g de peptone de blé, 5 g d'extrait de levure, 1 g de D-glucose H₂O,120 mg de FeCl₃,6H₂O, 400 mg de MgCl₂,6H₂O, 10 mg de CaCl₂,2H₂O, 6 g de NaCl, 5 mg de ZnCl₂, 6.8 g de (NH₄)₂SO₄ et 6 g de NH₄Cl, pH=6,5.

Selon un autre mode de réalisation avantageux, l'étape de pré-culture est mise en oeuvre par culture de la souche sur un milieu de pré-culture M0 liquide ou gélosé et qui comprend, et de préférence consiste en: 3,87 de peptone de blé ou 30g/L pour le milieu M0 liquide, 5 g d'extrait de levure, 1 g de D-glucose H₂O,120 mg de FeCl₃,6H₂O, 400 mg de MgCl₂,6H₂O, 10 mg de CaCl₂,2H₂O, 6 g de NaCl, 5 mg de ZnCl₂, 6.8 g de (NH₄)₂SO₄ et 6 g de NH₄Cl, pH= 6,5.

L'étape de pré-culture permet une amplification de l'inoculum. Elle peut être réalisée par ensemencement du milieu de pré-culture M0 avec une souche de *Staphylococcus aureus* par exemple à raison de 1.10⁷ CFU et incubation à 37°C pendant une durée pouvant varier par exemple de 5 heures à 20 heures dans une atmosphère contenant ou pas de 5 à 10 % en vol. de CO₂ (sans CO₂ pour le T5 et en présence de CO₂ dans le cas de l'utilisation de la souche Becker). L'étape de pré-culture peut afin d'augmenter la biomasse comprendre des pré-cultures successives dans des volumes croissants de milieu M0. La pré-culture sert ensuite à ensemencer le milieu liquide M1. Pour ce faire on peut utiliser une quantité d'inoculum telle que la DO680nm initiale soit de 0,2. Le volume de culture dans l'étape de pré-culture peut varier classiquement de 400 ml à 201. De préférence on maintient un rapport volume du milieu de pré-culture par rapport au volume du récipient inférieur ou égal à 20 %.

Après l'étape de culture les souches ainsi produites peuvent être récupérées par centrifugation et conservées sous forme de lyophilisats ou de congelats après addition de glycérol 20 % ou être utilisées directement pour ensemencer un plus grand volume de milieu de culture, ou encore être utilisées directement dans le procédé de production de polysaccharide capsulaire selon la présente invention.

Selon un mode de réalisation avantageux, le procédé de production tel que défini ci-dessus correspond à un procédé de production d'une souche Reynolds surproductrice.

Selon un autre mode de réalisation avantageux, le procédé de production tel que défini ci-dessus correspond à un procédé de production d'une souche CYL770 surproductrice.

La présente invention concerne également les souches surproductrices de *Staphylococcus aureus*, en particulier les souches Reynolds, Becker et CYL770, produites par le procédé tel que défini ci-dessus.
Les inventeurs ont réalisé un plan d'expérience qui montre que le milieu de culture M1 peut être simplifié et remplacé dans le cadre des procédés selon la présente invention par un milieu M2 tel que défini ci-dessous.
Selon un autre mode de réalisation, l'étape (a) utilise donc un milieu de culture M2 comprenant par litre de milieu de culture :
   - 52,5 à 100 g de peptone végétale, avantageusement de la peptone de blé,
   - 0 à 82,5 g de NaCl, avantageusement de 5g à 82,5g, en particulier 41g/l de NaCl
   - 0 à 15g/l d'un sel de magnésium, avantageusement de 0,01 à 15g/l, en particulier 15g/l de MgCl₂,6H₂O, et
   - 0 à 7g/l de sucre, avantageusement de 0,25 à 7g/l, en particulier 0.25g/l de D-glucose,H₂O,
   - dont le pH est compris dans une gamme de valeurs allant de 6.3 à 6.7.
Pour la définition des termes utilisés on se référera aux définitions données ci-dessus en relation avec le milieu de culture M1.
Les résultats du plan d'expérience montrent qu'il n'est pas nécessaire d'ajouter du sucre au milieu de culture M2 lorsque la peptone végétale contenait une teneur en sucres totale d'au moins 5%. Celle-ci peut comprendre un mélange de sucres tels que: fructose, glucose, mannotriose, raffinose, saccharose, stacchyose). Si tel n'est pas le cas il est alors nécessaire de rajouter au milieu de culture une autre source de sucre. Le sucre peut alors être rajouté à raison de 0,25g/l à 7g/l.
Selon un mode de réalisation avantageux, le milieu de culture M2 comprend 0,25g/l de sucre, en particulier de D-glucose, H₂O.
Le milieu de culture M2 peut avantageusement contenir un sel de magnésium sélectionné dans le groupe consistant en MgCl₂ et Mg SO₄. Les inventeurs ont mis en évidence que l'addition d'un sel de magnésium permet d'augmenter la robustesse du milieu de culture (les plages de variation des constituants sont sensiblement moins larges en présence de MgCl₂). Le chlorure de magnésium, en particulier MgCl₂,6H₂O, peut donc être ajouté avantageusement au milieu M2 dans une gamme de 0,01 g/l à 15g/l de milieu de culture. La meilleure robustesse est observée lorsque le milieu M2 contient 15g/l de MgCl₂,6H₂O. Des concentrations supérieures en sel de magnésium n'entraînent pas une augmentation supplémentaire de la robustesse.
Les inventeurs ont également mis en évidence que la productivité améliorée en polysaccharides est observée avec le milieu M2 dans une large gamme de concentrations en NaCl. En effet, le NaCl peut soit être absent, soit être utilisé dans une gamme de concentration allant de 5g/l à 100 g/l, avantageusement de 5g/l à 82,5g/l de milieu de culture.
Le plan d'expérience a par ailleurs permis de montrer qu'il y avait une forte interaction entre la concentration en peptone et la concentration en NaCl. Des gammes de concentrations pouvant être avantageusement utilisées dans le cadre des procédés selon la présente invention ont ainsi pu être définies.
Selon un mode de réalisation particulièrement avantageux, le milieu M2 tel que défini ci-dessus comprend 52,5 à 70 g de peptone végétale, avantageusement de la peptone de blé, et
5 à 37g de NaCl.
Selon un autre mode de réalisation particulièrement avantageux, le milieu M2 tel que défini ci-dessus comprend 66 à 82,5 g de peptone végétale, avantageusement de la peptone de blé, et 22 à 74g de NaCl.
Selon un autre mode de réalisation particulièrement avantageux, le milieu M2 tel que défini ci-dessus comprend 60 à 90,5 g de peptone végétale, avantageusement de la peptone de blé, et 35 à 63,5g de NaCl.
Selon un autre mode de réalisation particulièrement avantageux, le milieu M2 tel que défini ci-dessus comprend 79 à 95 g de peptone végétale, avantageusement de la peptone de blé, et 46 à 82,5g de NaCl.
Les milieux M2 selon ces 4 modes de réalisation peuvent avantageusement contenir 0,25g/l de glucose, en particulier D-glucose et 15g/l de MgCl2,6H₂O.
Selon un mode de réalisation particulier le milieu de culture M2 comprend, et avantageusement consiste en, 93g/l de peptone de blé, 41g/l de NaCl, 0,25g/l de D-glucose, H₂O, et 15g/l de MgCl₂,6H₂O, dont le pH est dans une gamme de valeurs de 6.3 à 6,7. Un tel milieu conduit par exemple à un rendement de 40 mg/l de polysaccharide T5 à partir d'une souche Reynolds.
Les inventeurs ont également montré par un plan d'expérience qu'un milieu M2 tel défini ci-dessus dans lequel la peptone végétale est remplacée par 65 à 85g, avantageusement 75g/l d'extrait de levure pouvait être utilisé dans les procédés selon la présente invention. Ce milieu est appelé milieu de culture M3.Le milieu de culture M3 selon la présente invention comprend donc par litre de milieu de culture :
- 65g/l à 80g/l d'extrait de levure, avantageusement 75g/l d'extrait de levure,
- 0 à 82,5 g de NaCl, avantageusement de 5g à 82,5g, en particulier 41g/l de NaCl
- 0 à 15g/l d'un sel de magnésium, avantageusement de 0,01 à 15g/l, en particulier 15g/l de MgCl₂,6H₂O, et
- 0 à 7g/l de sucre, avantageusement de 0,25 à 7g/l, en particulier 0.25g/l de D-glucose H₂O,
- dont le pH est compris dans une gamme allant de 6.3 à 6.7.

Des expériences supplémentaires ont montré que la gamme de peptone végétale (telle que définie ci-dessus) pouvait être étendue jusqu'à une valeur de 200g/l de milieu de culture. Les milieux M2 et M3, tels que définis ci-dessus, contenant une peptone végétale à raison de 52.5 à 200g/l sont donc inclus dans le cadre de la présente invention.

Le procédé de production de polysaccharide capsulaire comprend :
(a) la culture d'une souche de *Staphylococcus aureus* telle que définie ci-dessus sur un milieu de culture M1 ou M2 ou M3 tel que défini ci-dessus,
(b) l'inactivation de la culture ainsi produite, et
(c) la récupération du polysaccharide capsulaire.

L'étape (a) de culture du procédé de production de polysaccharide capsulaire peut être réalisée dans les mêmes conditions que celles décrites ci-dessus en relation avec l'étape (a) du procédé de production de souches surproductrices et peut également être précédée d'une étape de pré-culture telle que décrite ci-dessus.

La culture résultant de l'étape (a) est ensuite soumise à une étape d'inactivation.

L'étape d'inactivation peut être mise en oeuvre par traitement de la culture avec un mélange phénol/éthanol (V/V) à raison de 2% en volume au final, puis agitation par exemple à l'aide d'un barreau aimanté à température ambiante pendant 6 heures à 48 heures en fonction de la biomasse à traiter. L'inactivation est contrôlée par un test de mortalité. Pour ce faire, 100 microlitres de la culture traitée sont étalés sur une gélose Columbia 2 % NaCl et en parallèle 100 microlitres de la culture traitée sont ensemencés en bouillon trypcase soja additionné de 1/30^{ème} de sérum de cheval. Les cultures sont incubées pendant 48H à 37°C en position statique, les bouchons des flacons de bouillon restant dévissés. L"inactivation des cultures peut être contrôlée par une méthode comprenant une étape d'amplification en milieu TSB (sans ajout de sérum) sous agitation à 37°C pendant 16 à 18h, puis une boîte de pétri de gélose Columbia 2% NaCl est ensemencée avec 150µL de culture ainsi amplifiée. La boîte est elle-même placée à 37°C pendant 16 à 18h puis observée. Une absence de colonies témoigne de l'inactivation réussie de la culture traitée. L'absence de bactéries viables se traduit par une absence de pousse après 24 heures d'incubation à 37°C. Une fois que la mortalité de la culture est établie, on récupère le polysaccharide capsulaire. L'étape de récupération comprend une extraction du polysaccharide à partir des cellules. Différentes techniques d'extraction ont été décrites dans la littérature et peuvent être utilisées dans le cadre de la présente invention. On peut se référer par exemple aux articles de Lee J.C. Infect. Immunu. 1993 ; 67 :1853-1858 ; Dassy B. et al J. Gen. Microbiol. 1991 ; 137 :1155-1162. Une méthode d'extraction par autoclavage à 121°C est illustrée dans l'exemple 2.3. Une technique d'extraction utilisant la lysostaphine est décrite à l'exemple 2.5.

Dans le cas de la souche CYL 770, la plus grande partie du polysaccharide est secrétée dans le surnageant de culture et peut donc être récupérée directement à partir de ce dernier. Pour ce faire à l'issue de la culture, le milieu est centrifugé et le culot cellulaire est éliminé.
Le polysaccharide capsulaire résultant peut ensuite si nécessaire être purifié. Différentes techniques de purification ont été décrites dans la littérature et peuvent être utilisées dans le cadre de la présente invention. On peut se référer par exemple aux articles de Lee J.C. et al 1987 ; 55 :2191-2197 ; Fattom A. et al Infect. Immun. 1990 ; 58 :2367-2374. Une technique de purification est illustrée à l'exemple 2.5.

L'analyse de la composition molaire en monosaccharide et l'analyse par RMN du proton à 500MHz montrent que l'on obtient un polysaccharide dont la structure est conforme à celle décrite dans la littérature avec un taux de 0-acétylation supérieur à 70 % et un taux de pureté d'au moins 70 %. Les résiduels sont présents dans le produit final à raison d'environ 0,5 à 3% (p/p) de résiduels de nature protéique et moins de 0,1% (p/p) d'acides nucléiques résiduels et moins de 5% (p/p) d'acide lipotéchoique et d'acide techoïque résiduels.

La quantité de polysaccharide capsulaire obtenue peut être évaluée par la technique ELISA telle que décrite à l'exemple 2.4.

Le procédé de production de PS peut donc être utilisé avantageusement pour produire du polysaccharide T5, de préférence à partir d'une souche Reynold telle que CIP 103313 et du polysaccharide T8, de préférence à partir de la souche CYL770.

La présente invention concerne un milieu de pré-culture M0 tel que défini ci-dessus adapté à la culture de *Staphylococcus aureus.*

On entend dans le cadre de la présente invention par « milieu adapté à la culture de *Staphylococcus aureus »* un milieu qui permet d'obtenir d'une part une augmentation de la biomasse d'un facteur d'au moins 10 telle que déterminée par la mesure de la densité optique à 680 nm et d'autre part une viabilité telle que le rapport DO/CFU soit d'au moins 1 Unité de DO-10⁸CFU/ml. Pour déterminer ce rapport DO/CFU, la suspension bactérienne dont la densité optique a été mesurée à l'aide d'un spectrophotomètre est diluée dans du tampon PBS en utilisant des dilutions de raison 10. Le nombre de CFU contenues dans ces dilutions est évalué après ensemencement de ces dernières sur une boîte de pétri contenant un milieu nutritif (tel que de la gélose Colombia). Après incubation 24 heures à 37°C, les bactéries viables sont comptées et la correspondance entre le nombre de bactéries viables exprimé en Unité formant des colonies (CFU) et la densité optique (DO) peut ainsi être établie.

On entend dans le cadre de la présente invention par « milieu adapté à la surproduction de polysaccharide capsulaire » un milieu conduisant à une augmentation de la capacité de sécrétion en polysaccharide capsulaire d'une souche de *Staphylococcus aureus* qui se traduit par une production de polysaccharide capsulaire, telle que déterminée par un test ELISA tel que défini dans l'exemple 2.4 au moins 3 fois, de préférence au moins 5 fois et en particulier au moins 10 fois supérieure à celle produite par la culture de la même souche de départ dans les mêmes conditions de culture mais sur le milieu de Poutrel .

Dans le cas des souches recombinantes, on considère que le milieu est adapté à la surproduction de polysaccharide capsulaire s'il conduit à une augmentation de la capacité de sécrétion en polysaccharide capsulaire d'une souche de *Staphylococcus aureus* recombinante qui se traduit par une production de polysaccharide capsulaire, telle que déterminée par un test ELISA tel que défini à l'exemple 2.4, au moins 2 fois supérieure à celle produite par la culture de la même souche de départ dans les mêmes conditions de culture mais sur le milieu TSB.

### Exemple 1 : Préparation des milieux de culture

Dans un premier temps, des solutions mères de chacun des composants sont préparées. Pour ce faire les composants sont pesés sur une balance de précision et dissous en eau ultra filtrée (sauf indication contraire). Les solutions ainsi obtenues sont ensuite filtrées sur stéricup 0,22 micron et stockées à + 4°C à l'abri de la lumière. Les solutions mères de peptones de blé, d'extrait de levure et de FeCl₃, sont préparées extemporanément.

### 1.1- Milieu de Poutrel

Le milieu de Poutrel est préparé comme indiqué dans article de Poutrel et al., (Poutrel, B et al. (1995). Clin Diagn Lab Immunol 2, 166-171) par mélange des différentes solutions mères. Le pH est ensuite ajusté à 7 par ajout de NaOH 30% puis le milieu est filtré sur 0,22 micron.

La composition finale en g/l de milieu est : L-Cystine : 0,24g/L ; L-Aspartique acide : 2,4g/L ; L-Glutamique acide : 2,4g/L ; L-Proline : 2,4g/L ; L-Arginine : 0,36g/L ; L-Glycine : 2,4g/L ; L-Histidine : 0,48g/L ; L-Lysine HCl : 0,6g/L ; L-Sérine : 2,4g/L ; L-Valine : 0,48g/L ; L-Tyrosine : 0,18g/L ; L-Thréonine : 2,4g/L ; L-Alanine: 2,4g/L ; L-Isoleucine: 0,6g/L ; L-Leucine : 0,6g/L ; L-Phénylalanine : 0,2g/L ; L-Tryptophane : 0,06g/L ; L-Méthionine : 0,18g/L ; D-Glucose : 10g/L ; Acide nicotinique : 11,94 mg/L ; Thiamine HCl : 0,6 mg/L ; Ammonium Sulfate : 6,84g/L ; Potassium dihydrogénophosphate: 1,34 g/L ; Sodium diphosphate : 14,38 g/L ; Calcium chlorure : 5 mg/L ; Cobalt chlorure hyexahydraté : 2,4 mg/L ; Sulfate de cuivre: 0,5 mg/L ; Sulfate de manganèse : 5,6 mg/L ; sodium chlorure : 5,8 mg/L ; Chlorure de zinc : 3,4 mg/L ; Chlorure de fer : 27 mg/L ; Magnésium sulfate : 247 mg/L.

### 1.2- Les milieux M0 gélosé, M0 liquide, M1

### Pour le milieu M0 gélosé :

Dans un premier temps 150 ml de base gélosée sont préparés par dissolution de 3g d'agar dans 150mL d'H₂O ultra filtrée. La composition résultant est placée au bain-marie bouillant jusqu'à disparition des grains d'agar ( environ 30min) puis stérilisée à l'autoclave 30 min à 116 °C.
Le milieu M0 gélosé est ensuite préparé par mélange des différentes solutions mères dans l'ordre indiqué dans la composition ci-dessous), filtration du mélange résultant sur stéricup 0.22 micron et addition du mélange ainsi obtenu sous 50mL à la base gélosée de façon à obtenir un milieu contenant en g par litre :
peptone de blé : 3,87 g ; extrait de levure : 5 g ; D-glucose H₂O : 1 g ; NaCl : 6 g ;
MgCl₂,6H₂O,: 0,4 g ; (NH₄)₂SO₄: 6,8 g ; NH₄Cl: 6 g ; FeCl₃,6H₂O,: 120 10⁻³ g ; ZnCl₂ : 5 10-3 g ; CaCl₂,2H₂O: 10 10-3 g.

### 8 boîtes de pétri sont ensuite coulées.

La peptone de blé qui a été utilisée correspond à la peptone de blé d'Organotechnie (ref 19559). L'extrait de levure utilisé correspond à l'extrait de Beckton Dickinson (ref 212750) :

### Pour le milieu M0 liquide :

Le milieu M0 liquide est préparé par mélange des différentes solutions mères dans l'ordre indiqué dans la composition ci-dessous et filtration du mélange résultant sur stéricup 0.22 micron de façon à obtenir un milieu contenant en g par litre :
peptone de blé : 30 g ; extrait de levure : 5 g ; D-glucose H₂O : 1 g ; NaCl : 6 g ;
MgCl₂,6H₂O: 0,4 g ; (NH₄)₂SO₄: 6,8 g ; NH₄Cl: 6 g ; FeCl₃,6H₂O,: 120 10⁻³ g ; ZnCl₂ : 5 10-3 g ; CaCl₂,2H₂O : 10 10-3 g ; pH=6,5.

### Pour le milieu M1

Le milieu M1 est préparé par mélange des différentes solutions mères dans l'ordre indiqué dans la composition ci-dessous et filtration du mélange résultant sur stéricup 0.22 micron de façon à obtenir un milieu contenant en g par litre :
peptone de blé : 30 g ; extrait de levure : 5 g ; D-glucose H₂O : 1 g ; NaCl : 58,5 g ;
MgCl₂,6H₂O,: 0,4 g ; (NH₄)₂SO₄: 6,8 g ; NH₄Cl: 6 g ; FeCl₃,6H₂O,: 120 10⁻³ g ; ZnCl₂ : 5 10-3 g ; CaCl₂,2H₂O : 10 10-3 g

### 1. 3- Gélose Columbia 2% NaCl

Pour 1 litre de milieu, 44g de Columbia « Blood Agar Base » (Difco ref. 279240) sont pesés, puis on ajoute 500 mL H₂O ultra filtrée stérile. Le mélange obtenu est incubé au bain-marie à 100°C pendant environ 30 minutes (jusqu'à complète dissolution et obtention d'une solution limpide). 500 ml H₂O ultra filtrée stérile sont ensuite ajoutés. La solution est répartie en flacons Schotts de 200ml, puis stérilisée par autoclave à 116°C pendant 30 minutes. Les milieux sont ensuite maintenus à 55°C (bain-marie) avant d'être coulés en boîtes de pétri (25mL/boîte). Avant de couler les boîtes, on ajoute 13,3 mL de NaCl 30% (filtré 0,22µm) par flacon de 200mL pour obtenir une concentration finale de 2% (=2 g/100ml)

### 1.4- Tryptic Soy Agar (GTS)

Pour 1 litre de milieu, 40g de « Tryptic Soy Agar » (Difco ref. 236950) sont pesés, puis on ajoute 500mL H₂O ultra filtrée stérile. Le mélange obtenu est incubé au bain-marie à 100°C pendant environ 30 minutes (jusqu'à complète dissolution et obtention d'une solution limpide). 500 ml H₂O ultra filtrée stérile sont ensuite ajoutées. La solution est répartie en flacons Schotts de 200ml, puis stérilisée par autoclave à 116°C pendant 30 minutes. Les milieux sont ensuite maintenus à 55°C (bain-marie) avant d'être coulés en boîtes de pétri (25mL/boîte).

### 1.5- Bouillon "Bacto Trxptic Soy" Broth (TSB)

Pour 1 litre de milieu, 30g de « Bacto Tryptic Soy » ( Difco Ref. 211825) sont pesés, puis on ajoute 500mL H₂O ultra filtrée stérile. Le mélange obtenu est incubé au bain-marie à 100°C pendant environ 30 minutes (jusqu'à complète dissolution et obtention d'une solution limpide). 500 ml H₂O ultra filtrée stérile sont ensuite ajoutées. La solution est répartie en flacons Schotts de 200ml, puis stérilisée par autoclave à 116°C pendant 30 minutes. Les milieux sont ensuite maintenus à 55°C (bain-marie) avant d'être coulés en boîtes de pétri (25mL/boîte).

### Exemple 2 : Evaluation de la production de polysaccharide capsulaire de type 5 (CP5) dans différentes conditions de culture

### 2.1- Culture de la souche Reynolds

Des congelats ont été préparés de deux façons différentes selon que l'on souhaite se placer dans des conditions sans composés d'origine animale ou dans des conditions standards. Pour les conditions standards, un lyophilisat d'origine est repris sur une gélose Colombia+2% NaCl . Cette dernière est cultivée 18 heures à 37°C avec ou sans CO₂ en fonction de la souche. La culture est de nouveau repiquée sur une nouvelle boîte de gélose pendant 4 heures à 37°C pour obtenir des germes en phase exponentielle de croissance. La culture est alors récupérée dans du bouillon trypticase soja auquel est rajouté 20% de glycérol. Dans le cas où les conditions exemptes de composés d'origine animale sont requises, la pré-culture est réalisée sur milieu M0 18 heures à 37°C puis l'inoculum sert à repiquer un milieu liquide M0. La congélation se fait également après addition de glycérol 20%.
Un congelat Reynolds de 100µL obtenu par la première méthode citée ci-dessus est repris dans 900µL de PBS.
On ensemence deux boîtes de pétri de milieu M0 gélosé et deux boîtes de pétri de gélose columbia 2% NaCl en nappe avec 150µL de suspension bactérienne par boîte et on incube 18-20h à 37°C.
Les nappes sont raclées à l'oese et resuspendues dans 5mL de PBS. La DO₆₈₀ₙₘ des suspensions est mesurée, et permet de déterminer le volume de pré-culture nécessaire pour ensemencer les erlens de 50mL à DO₆₈₀ₙₘ=0,2.
La pré-culture sur gélose Columbia 2% NaCl est utilisée pour ensemencer un erlen de milieu de Poutrel, et la pré-culture sur M0 gélosé ( 18-20H à 37°C) est utilisée pour ensemencer un erlen de milieu M0 liquide et un erlen de milieu M1. Les volumes sont variables en fonction des essais, le plus souvent - 50mL. Pour maintenir une bonne oxygénation, le volume de milieu est inférieur ou égal à 20% du volume de l'erlen. Après vérification des DO₆₈₀ₙₘ initiales, les erlens sont placés sous agitation (x100tpm) à 37°C.
Le suivi des cultures se fait par mesure de la DO à 680nm à tₒ, t₈ₕ, t₂₄ₕ, t₃₂ₕ, t₄₈ₕ, t₅₆ₕ, et t₇₂ₕ, le pH initial est mesuré ainsi qu'à 72h de culture. Les résultats obtenus sur les cultures de 50 ml sont consignés dans les tableaux 1 et 2 ci-dessous.

**Tableau 1 :**

| Souche | Milieu de pré-culture | DO/CFU pour DO680=1 | Ecart type |
|---|---|---|---|
| Reynolds | gélose Columbia 2% NaCl | 5,3E+08 | 8,2E+07 |
| Reynolds | M0 gélosé | 4,2E+08 | 1,7E+08 |

**Tableau 2 : Mesure de la DO 680nm**

| Temps en heures | Poutrel | M0 liq. | M1 |
|---|---|---|---|
| 0 | 0,2 | 0,2 | 0,2 |
| 8 | 2,8 | 2,6 | 2,7 |
| 24 | 6,7 | 6,2 | 4,0 |
| 32 | 9,1 | 8,0 | 4,7 |
| 48 | 10,1 | 8,4 | 6,1 |
| 56 | 8,6 | 8,7 | 6,4 |
| 72 | 8,1 | 8,6 | 5,7 |
| pH initial | 7 | 6,5 | 6,5 |
| pH final | 8,5 | 8,5 | 8,5 |

### 2.2- Inactivation de la culture

A 72h les cultures sont réparties dans 4 flacons schott de 1L contenant chacun 750mL de culture.
On ajoute 15mL de phénol/éthanol (v/v) pour 750mL de culture soit 2% final et on agite le mélange à l'aide d'un barreau aimanté à température ambiante pendant 48H environ. Le test de mortalité est réalisé de la façon suivante :
100µL de culture traitée sont étalés sur gélose Columbia 2% NaCl et
100µL de culture traitée sont ensemencés en bouillon trypcase soja + 1/30^{e} sérum de cheval, puis on incube pendant 48H à 37°C (les bouchons des flacons de bouillon trypcase soja sont dévissés). Les cultures ainsi inactivées sont utilisées pour extraire le polysaccharide et évaluer la quantité produite par ELISA.

### 2.3- Extraction du polysaccharide produit

Les cultures de 72H inactivées sont ensuite centrifugées 30min à 3500rmp à +4°C, les surnageants de culture sont séparés et stockés à +4°C. Les culots sont repris dans 5mL de PBS et autoclavés 60min à 121°C puis centrifugés 30min à 25000g +4°C. Le surnageant (extrait 1) est récupéré. Les culots sont repris dans 5mL de PBS, autoclavés 60min à 121°C et centrifugés 30min à 25000g +4°C. Le surnageant (extrait 2) est récupéré. Les extraits 1 et 2 sont rassemblés et traités à la protéinase K 50 microgrammes/ml 1H à 37°C puis la protéine est inactivée 20 minutes à 75°C.

### 2.4- Evaluation de la quantité de polysaccharide produite par ELISA

Les sera polyclonaux dirigés contre la capsule de type 5 ou de type 8 ont été produits selon la méthode décrite par Karakawa et al. J Clin Microbiol. 1985 Sep;22(3):445-7. Les monoclonaux utilisés ont été produits par le protocole tel que décrit par Hochkeppel et al. J Clin Microbiol. 1987 Mar;25(3):526-30 et nous ont été transmis par l'Institut Pasteur

La quantité de polysaccharide capsulaire produite est déterminée par ELISA. Pour ce faire, un test ELISA en sandwich est réalisé sur des plaques de 96 puitss (Nunc, Roskilde, Danemark). Dans un premier temps on dépose dans les puitss un anticorps monoclonal anti-CP5 ( 1 microgramme par ml dans du tampon carbonate de sodium 0,05 M pH=9,6 (sigma) - 100 ng/puits), les plaques sont incubées toute la nuit à 4°C. Les plaques sont ensuite lavées 4 fois avec 300 microlitres par puits de tampon PBS (Eurobio) additionné de 0,05% de tween 20 (Sigma) (PBS-Tween) puis saturées pendant une heure à 37°C avec 250 microlitres par puits de PBS-Tween additionné de 1% (vol/poids) de BSA (Eurobio) (PBS-Tween-BSA). Les plaques sont ensuite lavées à trois reprises avec la solution PBS-Tween.
Des dilutions en série d'un facteur 2 des extraits de l'étape 2.3 par utilisation de la solution PBS-Tween-BSA sont réalisées et ajoutées à raison de 100 micro litres par puits. Les plaques sont incubées pendant 90 minutes à 37°C puis lavées 4 fois avec du PBS-Tween. Des anticorps polyclonaux de lapin anti-CP5 produits selon le protocole de Karakawa (Karakawa et al., 1985, J. Clin. Microbiol. 1985, Sep ; 22(3) : 445-7) sont dilués dans du PBS-Tween-BSA (1/2000) puis ajoutés aux puitss à raison de 100 microlitres par puits, et on incube pendant 90 minutes à 37°C. Les plaques sont ensuite lavées 4 fois avec du PBS-Tween. Le conjugué IgG de chèvre anti-lapin - peroxydase (Southern biotech.), dilué dans du PBS-Tween-BSA (1/6000) est ajouté à raison de 100 microlitres par puits et on incube pendant 1 heure à 37°C. Les plaques sont lavées 8 fois avec 300 microlitres de PBS-Tween par puits et on ajoute 100 microlitres par puits d'une solution de substrat TMB (TEBU) puis on incube dans le noir pendant 15 min à température ambiante (environ 22°C). La réaction enzymatique est stoppée par addition de 100 microlitres de H₃PO₄ 1M (sigma) par puits.
Un lecteur de plaque automatique (Versamax) mesure ensuite la densité optique à 450 nm. La valeur du témoin est soustraite et la quantité évaluée par comparaison avec une courbe étalon.
Les résultats sont consignés dans le tableau 3 ci-dessous.

**Tableau 3 :**

| | | | | | Dosage ELISA CP5 (X3) (dans le culot) | |
|---|---|---|---|---|---|---|
| Milieu de culture | Pré-culture | Vol. culture (ml) | DO680 Initiale | DO680 Finale | Moyenne (microg/ml de culture) | Ecart type |
| Poutrel | Gel.Col. 2% NaCl | 50 | 0,2 | 8,1 | 3,6 | 0,3 |
| M0 liquide | M0 gélosé | 50 | 0,2 | 8,6 | 9,7 | 1,2 |
| M1 | M0 gélosé | 50 | 0,2 | 5,7 | 36,0 | 1,5 |

### 2.5- Extraction et purification du polysaccharide CP5

La souche Reynolds est cultivée comme cela est décrit au point 2.1 selon un protocole comprenant une pré-culture sur milieu M0 gélosé puis une culture dans 400 ml de milieu M1 dans des erlens. Les cultures sont regroupées pour obtenir 3 litres de culture à partir desquels on extrait et purifie le polysaccharide T5 produit.
L'intégralité de la culture est inactivée par traitement au phénol-éthanol (V/V 2% final) puis centrifugée 30mn à 25000g. Un culot d'environ 10,9 g de germes inactivés humides par litre de culture est obtenu.
Les germes sont mis en suspension à raison de 0,5 g /ml de Tris 50mM, MgSO₄2 mM, pH 7,5. Une solution aqueuse de lysostaphine à 5 mg/ml est ajoutée de façon à obtenir une concentration finale de 100µg/ml. Après une incubation de 4 heures à 37 °C sous agitation magnétique, on ajoute une solution de benzonase à 5 U / ml final et l'incubation s'effectue pendant 2 heures à 37°C sous agitation magnétique.
Après centrifugation (25000g, 30mn), les surnageants d'extraction sont récoltés, poolés et ultrafiltrés. Le surnageant est préalablement dilué au 1/4 en H₂O pour obtenir un volume suffisant pour le système d'ultrafiltration. L'ultrafiltration est réalisée sur 2 cassettes UF 50 kD dans les conditions suivantes :H₂O : 10 volumes ; puis Tris 50 mM pH 8,0 : 3 volumes. Le volume de retentat reste constant au cours de l'ultrafiltration. Le retentat constitue ce qui est appelé l'extrait CP5.
L'extrait CP5 est incubé à 37°C en présence de MgCl₂ (1mM en final) et de benzonase (5U/ml final soit ∼ 40U par gramme de germes humides traités) sous agitation. Après 3 heures d'incubation, on ajoute la benzonase à une concentration finale de 5U/ml, l'incubation est poursuivie pendant 3 heures. Une solution aqueuse de pronase à 10mg/ml est ajoutée de façon à obtenir une concentration finale de 4 U / ml (soit ∼ 30U par gramme de germes humides traités), additionnée de CaCl₂à une concentration finale de 1mM. Le mélange est alors incubé pendant 15 heures à 37°C. Une ultrafiltration 50 kD est réalisée afin d'éliminer les fragments d'acides nucléiques et de protéines sur 2 cassettes UF 50KD dans les conditions suivantes : H₂O : 10 volumes, puis Tris 50mM pH 8,0 : 3 volumes. Le volume de retentat reste constant au cours de l'ultrafiltration. Au retentat précédemment obtenu est ajouté : MgCl₂1mM en final, Benzonase 0,1U/ml en final, et on incube 3 heures sous agitation magnétique.
Une solution aqueuse de pronase à 10 mg/ml est ajoutée à une concentration finale de 0,1 U /ml, additionnée de CaCl₂à une concentration finale de 1mM, puis on incube pendant 2 heures à 37°C.
Une ultrafiltration sur 2 cassettes UF 30KD est alors effectuée dans les conditions suivantes : H₂O : 10 volumes, puis Tris 50mM pH 7,5 : 3 volumes. Le retentat obtenu présente un précipité / trouble blanchâtre qui est éliminé par centrifugation à 8000g / 30 min.
Une étape de purification sur chromatographie d'échange d'ions est ensuite réalisée. Elle permet l'élimination des résiduels notamment l'acide teichoïque (TA) et l'acide lipoteichoïque (LTA).
Les conditions suivantes sont utilisées : trois colonnes Hi Trap Q HP de 5 ml sont connectées en série (Réf: Amersham 17-1154-01) ; Tampon d'équilibration : Tris 20mM pH 7,5 ; Débit : 2ml/mn ; Injection de l'échantillon en tampon d'équilibration.

### Elution :

- Tris 20mM PH 7,5 : 5 volumes de colonne
- gradient Tris 20mM PH 7,5 NaCl 0-0,5M : 10 volumes de colonne
- Tris 20mM, NaCl 0,5M PH 7,5 : 5 volumes de colonne
- gradient Tris 20mM PH 7,5 NaCl 0,5-1M : 5 volumes de colonne
- Tris 20mM, NaCl 1 M PH 7,5 : 5 volumes de colonne
- Tris 20mM PH 7,5 : 5 volumes de colonne
L'élution est suivie par mesure de l'absorbance à 206 et 280nm. Une analyse par CZE permet d'identifier les différents composants élués au cours de la chromatographie.
Le polysaccharide CP5 est élué à une concentration en NaCl comprise entre 0,2 M et 0,3 M. Dans les conditions chromatographiques utilisées, celui ci est bien séparé des résiduels. Notamment l'acide teichoïque qui est élué entre 0,36 M et 0,48 M en NaCl et l'acide lipoteichoïque qui est élué entre 0,74 M et 0,86 M en NaCl.
Les fractions contenant le CP5 sont rassemblées et dialysées à +4°C contre 20 volumes d'une solution aqueuse de NaCl 0,5 M (2-3 bains) puis contre de l'eau ultrafiltrée 20 volumes (3-4 bains). Le Tris est ainsi éliminé.
Le produit purifié est lyophilisé et conservé à -20°C.
A partir de 3 litres de culture, on obtient 74 mg de polysaccharide capsulaire T5 en fin de purification. Les caractéristiques du produit sont consignées dans le tableau 4 ci-dessous.

**Tableau 4 :**

| | Dosage CP5 | O-acétyl (CP en poids de poudre) | | O-acétyl (CP dosage CE) | | protéines | ADN |
|---|---|---|---|---|---|---|---|
| Méthode analytique | RMN | colorimétrie | | | | Micro BCA direct | Picogreen |
| Unités | % par rapport au poids de poudre | Mmoles /mgCP | Moles/mole UR | Mmoles/mg CP | Moles/moles UR | % P/P par rapport poids de poudre | % P/P par rapport poids de poudre |
| Résultats | 80% | 1,32 | 0,87 | 1,65 | 1,08 | 2,7% | <0,1% |

### Exemple 3 : Evaluation de la productivité de polysaccharides capsulaires de type 8 (CP8) dans différentes conditions de culture

L'évaluation est réalisée sur les souches Becker et CYL770.

### 3.1- Culture de la souche Becker

Les congelats sont preparés de deux façons différentes selon que l'on souhaite se placer dans des conditions sans composés d'origine animale ou dans des conditions standards. Pour les conditions standards, un lyophilisat d'origine est repris sur une gélose Colombia+2% NaCl. Cette dernière est cultivée 18 heures à 37°C avec ou sans CO₂ en fonction de la souche. La culture est de nouveau repiquée sur une nouvelle boîte de gélose pendant 4 heures à 37°C pour obtenir des germes en phase exponentielle de croissance. La culture est alors récupérée dans du bouillon trypticase soja auquel est rajouté 20% de glycérol. Dans le cas où les conditions exemptes de composés d'origine animale sont requises, la pré-culture est réalisée sur milieu M0 18 heures à 37°C puis l'inoculum sert à repiquer un milieu M0 liquide. La congélation se fait également après addition de glycérol 20%.
Un congelat de 100µl Becker préparé selon la méthode en bouillon trypticase soja est repris dans 900µl de PBS. Trois boîtes de pétri de M0 gélosé, deux boîtes de pétri de gélose columbia 2% NaCl, et une boîte de pétri GTS sont ensemencées en nappe avec 150µL de suspension bactérienne par boîte. Les boîtes sont incubées pendant 18/20h à 37°C + 5% CO₂.
Les nappes sont raclées à l'oese et resuspendues dans 5mL de PBS .
La DO₆₈₀ₙₘ des suspensions est mesurée, et permet de déterminer le volume de pré-culture nécessaire pour ensemencer les erlens de 50mL à DO₆₈₀ₙₘ=0,2.
La pré-culture sur gélose columbia 2% NaCl est utilisée pour ensemencer un erlen de milieu de Poutrel, la pré-culture sur GTS est utilisée pour ensemencer un erlen de TSB, et la pré-culture sur milieu M0 gélosé est utilisée pour ensemencer un erlen de milieu de M0 liquide et un erlen de milieu M1.
Pour maintenir une bonne oxygénation, le volume de milieu est inférieur ou égal à 20 % du volume de l'erlen.
Après vérification des DO₆₈₀ₙₘ initiales, les erlens sont placés sous agitation x100tpm à 37°C.
Des expériences similaires sont réalisées dans des erlens contenant 400 ml de milieu de culture.

### 3.2- Culture de la souche CYL770

Pour la souche recombinante CYL770, la méthode de préparation des congelats utilise un repiquage du congelat initial sur milieu TSB ou sur un milieu M0 liquide. La culture s'effectue pendant 5 heures à 37°C sous agitation. La congélation est réalisée après addition de 20% de glycérol dans le milieu.
Un congelat de 1ml CYL770 ainsi obtenu est repris par 10ml de milieu M0 liquide puis placé en erlen de 50ml et incubé pendant 18/20h à 37°C sous agitation x100tpm. Un deuxième congelat CYL770 est repris dans 10mL de milieu TSB puis placé sous agitation dans les mêmes conditions que précédemment.
La DO₆₈₀ₙₘ des pré-cultures liquides est mesurée, et permet de déterminer le volume de pré-culture nécessaire pour ensemencer les erlens de 50ml à DO₆₈₀ₙₘ=0,2.
La pré-culture en TSB permet d'ensemencer un erlen de TSB, la pré-culture en M0 liquide permet d'ensemencer un erlen en milieu M1. Pour maintenir une bonne oxygénation, le volume de milieu est inférieur ou égal à 20 % du volume de l'erlen. Après vérification des DO₆₈₀ₙₘ initiales, les erlens sont placés sous agitation x100tpm à 37°C.
Des expériences similaires sont réalisées dans des erlens contenant 400 ml de milieu de culture.

### 3.3- Suivi des cultures

Le suivi des cultures se fait par mesure de la DO à 680 nm à tₒ, t₈ₕ, t₂₄ₕ, t₃₂ₕ, t₄₈ₕ, t₅₆ₕ, et t₇₂ₕ.
Le pH initial est mesuré ainsi qu'à 72h de culture. Les résultats des cultures de 50 ml sont consignés dans le tableau 5 ci-dessous.

**Tableau 5 :**

| | Becker | | | | CYL770 | |
|---|---|---|---|---|---|---|
| Temps en heures | Poutrel | M0 liquide | M1 | TSB | TSB | M1 |
| 0 | 0,3 | 0,3 | 0,3 | 0,2 | 0,2 | 0,2 |
| 8 | 3,3 | 3,2 | 3,6 | 5,7 | 3,3 | 2,3 |
| 24 | 7,4 | 8,0 | 4,9 | 7,1 | 5,6 | 3,4 |
| 32 | 7,8 | 8,6 | 5,9 | 7,4 | 5,9 | 4,0 |
| 48 | 8,3 | 8,6 | 6,3 | 7,5 | 4,7 | 5,8 |
| 56 | 8,1 | 8,5 | 5,3 | 5,7 | 3,9 | 5,1 |
| 72 | 7,0 | 7,5 | 6,6 | 5,8 | 2,9 | 5,4 |
| pH initial | 7 | 6,5 | 6,5 | 7 | 7 | 6,5 |
| pH final | 9 | 8,5 | 8,5 | 9 | 9 | 8,5 |

### 3.4- Inactivation des cultures

Les cultures sont inactivées selon le même protocole que celui décrit au point 2.2 ci-dessus. L'extraction du polysaccharide produit est mise en oeuvre selon le même protocole que celui décrit au point 2.3 ci-dessus.

### 3.5- Evaluation de la quantité de polysaccharide produite par ELISA

L'évaluation de la quantité de polysaccharide produite est réalisée par utilisation du même test ELISA que celui décrit au point 2.4 ci-dessus, sauf que les anticorps utilisés sont des anticorps anti-CP8 et le test est réalisé sur les extraits de culots et sur le surnageant de culture. L'adsorption a été réalisée avec un ascite contenant le monoclonal K8-24 dilué au 1/20000^{e,} et l'anticorps polyclonal de lapin est un sérum adsorbé dilué au 1/1000^{e}.
Les résultats obtenus pour des cultures de 50 ml et de 400 ml sont consignés dans le tableau 6 ci-dessous. Pour les cultures de 400ml l'analyse est faite à chaque fois sur un seul échantillon de 50 ml d'une culture et la quantité totale est ensuite calculée et reportée dans le tableau 6.

**Tableau 6 :**

| | | | Dosage ELISA du CP8 (X2) | | | |
|---|---|---|---|---|---|---|
| | | | culot | | Surnageant | |
| Souche | Culture | Pré-culture | Moyenne (microg/ml de culture) | Ecart type | Moyenne (microg/ml de culture) | Ecart type |
| Becker (50 ml) | Poutrel | Gel. Col. 2% NaCl | 1,3 | 0 | 1,8 | 0 |
| | M0 liq. | M0 gel. | 1,9 | na | 3,6 | na |
| | M1 | M0gel. | 14,0 | 1,6 | 43,2 | 2,2 |
| | TBS | GTS | 0 | 0 | 0 | 0 |
| Becker (400 ml) | M1 | M0 gel. | 23,04 | | 57,6 | |
| CYL770 | M1 | M0liq. | 8,7 | 0,5 | 534,2 | 75,2 |
| (50 ml) | TSB | TSB | 4,7 | 1,3 | 244 | na |
| CYL770 | M1 | M0liq. | 27,18 | na | 579,6 | na |
| (400 ml) | TSB | TSB | 3,6 | na | 216 | na |

| | | | | | | |
|---|---|---|---|---|---|---|
| na: non applicable | | | | | | |

### 3.6- Récupération et purification du PS produit

La récupération du polysaccharide T8 a été réalisée à partir des cultures en erlens de 400 ml de la souche Becker en milieu M-1 par utilisation du procédé tel que décrit à l'exemple 2 pour l'obtention du polysaccharide T5.
La purification du polysaccharide T8 a également été réalisée à partir des cultures en erlens de 400 ml de la souche CYL770 en milieu M-1 et des cultures sur milieu TSB. Pour ce faire 800 ml de culture sont inactivés par traitement au phénol-éthanol selon le procédé décrit ci-dessus, puis centrifugée 30 mn à 25000 g. 720 ml de surnageant ont été obtenus.
A 500 ml de ce surnageant est ajoutée une quantité d'acétate de sodium, de façon à obtenir une concentration finale de 4% en acétate de sodium. La suspension est mise sous agitation pendant 30 minutes à température ambiante. Puis, une quantité d'éthanol de façon à obtenir une concentration finale en éthanol de 50 % est ajoutée. La suspension est alors agitée à température ambiante pendant 30 mn, puis stockée sans agitation une nuit à +4°C.
Après centrifugation à 15000 g pendant 30 mn, le surnageant est récolté. A ce surnageant sont ajoutés de l'acétate de sodium et de l'éthanol de façon à obtenir une concentration finale de 4% en acétate de sodium et de 85 % en éthanol. La suspension est mise sous agitation 30 mn à température ambiante puis stockée sans agitation une nuit à 4°C. Après centrifugation à 15000 g pendant 30 mn, le culot est récolté.
Le culot ainsi obtenu est repris par 100 ml d'eau ultrafiltrée (concentration d'un facteur 5 par rapport au volume de surnageant de culture centrifugé). Après agitation magnétique jusqu'à dissolution complète du culot à température ambiante, la suspension obtenue est dialysée contre 2 litres d'eau ultrafiltrée avec trois changements de bain dans la journée à température ambiante, suivi d'une étape d'une nuit à 4°C. On ajoute ensuite à la suspension ainsi obtenue du Tris et du MgCl₂afin d'obtenir une concentration finale en Tris de 50 mM et de MgCl₂de 1 mM. Cette solution est incubée à 37°C en présence de benzonase (5U/ml final) sous agitation. Après 3 heures d'incubation, on ajoute de la benzonase à une concentration finale de 5 U/ml et l'incubation est poursuivie pendant 3 heures.
Une solution aqueuse de pronase à 10 mg/ml est ajoutée de façon à obtenir une concentration finale de 4 U / ml, additionnée de CaCl₂à une concentration finale de 1 mM. Cette étape est alors incubée pendant 15 heures à 37°C. Si apparaît un trouble/opalescence visible, une centrifugation à 8000 g pendant 30 min est effectuée. Une ultrafiltration 30 kDa est réalisée afin d'éliminer les fragments d'acides nucléiques et de protéines dans les conditions suivantes : H₂O : 10 volumes ; puis Tris 20mM pH 7,5 : 3 volumes.
Le volume de retentat reste constant au cours de l'ultrafiltration et obtention d'une solution limpide très légérement colorée.
Si le retentat obtenu présente un trouble / opalescence, celui ci est éliminé par centrifugation à 8000 g pendant 30 min.
Une étape de chromatographie échangeuse d'ions est ensuite réalisée pour compléter l'élimination des résiduels notamment l'acide teichoïque (TA) et l'acide lipoteichoïque (LTA) dans les conditions suivantes : Gel Q sepharose HP ; Colonne Hi Trap Q HP de 20 ml ; Tampon d'équilibration : Tris 20mM pH 7,5 ; Débit : 2 ml/mn ; Injection de l'échantillon en tampon d'équilibration. Injection de l'équivalent de 125 ml de surnageant de culture pour 20 ml de gel ; Elution :
- Tris 20mM pH 7,5 : 5 volumes de colonne
- gradient Tris 20mM pH 7,5 NaCl 0-0,5 M 20 volumes de colonne
- Tris 20mM, NaCl 0,5 M pH 7,5 : 5 volumes de colonne
- Tris 20mM, NaCl 1 M pH 7,5 : 5 volumes de colonne
- Tris 20mM pH 7,5 : 5 volumes de colonne
L'élution est suivie par mesure de l'absorbance à 206 et 280nm. Une analyse par CZE permet d'identifier les différents composants élués au cours de la chromatographie.

Nous avons ainsi pu déterminer que le polysaccharide CP8 est élué à une concentration en NaCl comprise entre 0,2 M et 0,3 M. Dans les conditions chromatographiques utilisées, celui ci est bien séparé des résiduels dont la quantité est infime après cette étape de chromatographie. Notamment l'acide teichoïque qui est élué entre 0,36 M et 0,48 M en NaCl et l'acide lipoteichoïque qui est élué entre 0,74 M et 0,86 M en NaCl.
Les fractions contenant le CP8 sont rassemblées et dialysées à +4°C contre 20 volumes d'une solution aqueuse de NaCl 0,5 M (2-3 bains) puis contre de l'eau ultrafiltrée 20 volumes (3-4 bains). L'étape en forte concentration de NaCL permet une bonne élimination du Tris.
Le produit ainsi purifié est lyophilisé et conservé à -20°C.
Les résultats obtenus sont consignés dans le tableau 7 ci-dessous.

**Tableau 7 :**

| | Dosage CP8 | O-acétyl (CP en poids de poudre) | | O-acétyl (CP dosage CE) | | protéines | ADN |
|---|---|---|---|---|---|---|---|
| Méthode analytique | CZE | colorimétrie | | | | Micro BCA direct | Picogreen |
| Unités | % par rapport au poids de poudre | Mmoles /mgCP | Moles/mole UR | Mmoles/mg CP | Moles/moles UR | % P/P par rapport poids de poudre | % P/P par rapport poids de poudre |
| Becker (P005) culot M0 liq./M1 | 71% | 0,93 | 0,61 | 1,31 | 0,86 | 2,2% | nd |
| CYL770 (P006) surnageant M0 liq./M1 | 85% | 1,03 | 0,68 | 1,22 | 0,80 | 0,5% | <0,1% |
| CYL770 (P007) surnageant TSB/TSB | 62% | 1,08 | 0,71 | 1,75 | 1,14 | 3,5% | nd |

| | | | | | | | |
|---|---|---|---|---|---|---|---|
| nd = non déterminé | | | | | | | |

### Exemple 4 : Culture de la souche de Staphylococcus aureus Reynolds type 5 en fermenteur de 30 litres.

### 4.1- Culture sur milieu BTS + NaCl

Le congelat de la souche Reynolds préparé par la première méthode du point 2.1 est inoculé à raison de 0,5% en Vol. dans deux erlens à chicanes de 2L (El et E2) contenant chacun 200mL de milieu Bouillon Tryptone Soja additionné de NaCl (55g/L). Les erlens sont incubés à 37°C pendant 5±1 heures (E1) ou 48 heures (E2) sous une agitation de175tpm.
Le fermenteur Braun de 30 litres contenant 20 litres du même milieu est inoculé avec 70 ml de pré-culture (E1) estimé à ∼1,38.10¹¹ CFU (∼5h de culture et DO₆₀₀ₙₘ finale dans l'erlen proche de 4). La culture est réalisée dans les conditions suivantes : Température : 37°C ; Durée de culture : 48±2 heures ; Régulation du pH à 6,50 avec NH₄OH 25% et H₃PO₄ 10% ; PO₂ 30% (régulation en cascade : agitation 20 à 80%, air de 20 (1vvm) à 60% (3vvm), enrichissement en oxygène de 0 à 100%) ; Pression : 100mbars ; Régulation antimousse par le struktol 1150^{ème}.
Le suivi de la culture est réalisé les premières heures avec des prélèvements réguliers, ainsi qu'à 20±2 heures de culture, 24±2 heures de culture, 42±2 heures de culture pour contrôler la DO à 600 nm ; déterminer le poids sec ; déterminer la viabilité ; observer la morphologie ; doser le glucose; doser l'acétate et déterminer la productivité en polysaccharide par le test ELISA sur 6 culots de DO=50, après 4±2 heures, 18±2 heures, 24±3 heures, 44±3 heures et 48±2 heures de culture.
Les cellules sont récoltées après 48±2 heures de culture dans des godets de centrifugation de 1L (12 godets/20L de culture) et centrifugées 20 minutes à 7000 g à 4°C. Le poids humide est déterminé après élimination du surnageant. Les culots sont conservés à ≤-20°C jusqu'à l'étape d'inactivation des germes au phénol/éthanol puis récupération et purification du polysaccharide T5 selon le procédé de l'exemple 2.
Le second erlen (E2) est suivi sur 48 heures afin de suivre la productivité de la capsule CP5 à 10h, 24h et 48h de culture.

### 4.2- Culture sur milieu de Poutrel

Le congelat de la souche *Staphylocoque aureus* Reynolds type 5 préparé selon la première méthode décrite au point 2.1 est inoculé à raison de 1% en Vol. dans deux erlens à chicanes de 2 litres (E1 et E2) contenant chacun 200 mL de milieu de Poutrel. Les erlens sont incubés à 37°C à 175 tpm pendant 16±2 heures. Les *pré-cultures* sont poolées et utilisées pour inoculer le fermenteur. La culture en fermenteur B. Braun de 30L contenant 20L de milieu de Poutrel est réalisée dans les conditions suivantes: Température: 37°C; Durée de culture : 48±2 heures; Régulation du pH à 6,50 avec NH₄OH 25% et H₃PO₄ 10%; PO₂ 30% (régulation en cascade: agitation 20 à 80%, air de 20 (1vvm) à 60% (3vvm), enrichissement en oxygène de 0 à 100%); Pression: 100mbars; Régulation antimousse par le struktol 1/50^{ème.}
La culture est suivie les premières heures ainsi qu' à 24±2 heures de culture et 30±2 heures de culture, avec des prélèvements pour contrôler la DO à 600 nm ; déterminer le poids sec ; déterminer la viabilité ; observer la morphologie ; doser le glucose ; doser l'acétate, doser les acides aminés libres et déterminer la productivité en polysaccharide par le test ELISA.
Les cellules sont récoltées après 48±2 heures de culture.
2L de la récolte sont récoltés dans un schott de 2 et traités en phénol/éthanol (v/v) à une concentration finale de 2% (V/V). Ce mélange est mis sous agitation pendant 6h±1 heures à 22°C±3°C. Après élimination du surnageant du reste de la culture, les culots bactériens sont repris avec un volume minimal de tampon PBS puis autoclavés pour inactivation et récupération du polysaccharide selon le procédé décrit dans l'exemple 2 ci-dessus.
Le reste de la culture est récolté dans des godets de centrifugation de 1 L (12 godets/18L de culture) et centrifugé 20 minutes à 7000 g à 4°C. Le poids humide est déterminé après élimination du surnageant. 2 litres de surnageant sont conservés à ≤-20°C après filtration 0,45µm et autoclavage 55 minutes à 121°C.
Les 12 culots sont remis en suspension dans un volume minimum de tampon PBS (environ 650mL) à l'aide de l'ultraturax. Les suspensions sont poolées, homogénéisées et réparties équitablement dans 3 schott de 500mL (environ 300mL/schott) avant autoclavage 55min à 121°C. Les suspensions autoclavées sont ensuite poolées et le polysaccharide est récupéré selon le protocole décrit dans l'exemple 2 ci-dessus.

### 4.3- Culture sur milieu Columbia + NaCl

Un congelat de *Staphylocoque aureus* Reynolds type 5 préparé selon la première méthode du point 2.1 est inoculé à raison de 1% en Vol. dans deux erlens à chicanes de 2L contenant chacun 200 mL de milieu columbia + 20g/l de NaCl(Difco ref 294420)1. Les erlens sont incubés à 37°C pendant 24±2 heures sous une agitation de 175 tpm. Après 24±2 heures, les pré-cultures sont poolées et utilisées pour inoculer le fermenteur. La culture dans le fermenteur de 30L B. Braun contenant 20 1 du même milieu est réalisée dans les conditions suivantes : Température : 37°C ; Durée de culture : 24±2 heures ; Régulation du pH à 7,00 avec NH₄OH 25% et H₃PO₄ 10% ; PO₂ 30% (régulation en cascade : agitation 20 à 80%, air de 20 (1vvm) à 60% (3vvm), enrichissement en oxygène de 0 à 100%) ; Pression : 100mbars ; Régulation antimousse par le struktol 1150^{ème}.
La culture est suivie régulièrement avec des prélèvements réguliers pour contrôler la DO à 600 nm ; déterminer le poids sec ; déterminer la viabilité ; observer la morphologie ; doser le glucose; doser l'acétate et déterminer la productivité en polysaccharide par le test ELISA.
Les cellules sont ensuite récoltées après 24±2 heures de culture. La culture finale est transférée dans des godets de centrifugation de 1 L (12 godets/20L de culture) et centrifugée 20 minutes à 7000 g à 4°C. Le poids humide est déterminé après élimination du surnageant. Après centrifugation, Les 12 culots sont remis en suspension à l'aide de l'ultraturax dans un volume minimum de tampon PBS compris entre 1 et 1,2 L. Les suspensions sont poolées, homogénéisées et réparties équitablement en schott de 500mL puis autoclavées 55 minutes à 121°C. Les suspensions autoclavées sont ensuite poolées et le polysaccharide est récupéré selon le protocole décrit dans l'exemple 2 ci-dessus.

### 4.4- Culture sur milieu M1

Un congelat de *Staphylococcus aureus* Reynolds type 5 préparé selon la première méthode décrite au point 2.1 ci-dessus est repris en tampon PBS 1C à raison de 1mL de congelat pour 9mL de tampon pour ensemencer environ 10 boîtes de milieu M0 gélosé à raison de 150µL/boîte.
Les boîtes sont incubées à 37°C pendant 15 à 18 heures. Après une incubation de 15 - 18h à 37°C, les nappes formées sont râclées avant d'être reprises en suspension en milieu M1 pour inoculer deux erlens de composition identique (200mL/2L- El et E2) de façon à avoir une DO₆₀₀ₙₘ initiale proche de 0,2 dans chaque erlen. Les erlens sont incubés à 37°C, sous une agitation de 175tpm, pendant environ 5h (culture en phase exponentielle E1), ou 72 heures (E2).
Une partie de la culture de El (DO₆₀₀ₙₘ proche de 3) est utilisée pour inoculer le fermenteur B. Braun de 30L contenant 20L de milieu M1 de façon à avoir une DO₆₀₀ₙₘ initiale proche de 0,02.
La culture est mise en oeuvre dans les conditions suivantes : Température : 37°C ; Durée de culture : 72±4 heures ; Régulation du pH à 6,50 avec NH₄OH 25% et H₃PO₄ 10% ; PO₂ 30% (régulation en cascade : agitation 20 à 80%, air de 20 (1vvm) à 60% (3vvm), enrichissement en oxygène de 0 à 100%) ; Pression : 100mbars ; Régulation antimousse par le struktol 1/50^{ème}
1L de culture est ensuite récolté dans des godets de centrifugation et centrifugé 20 minutes à 7000 g à 4°C afin de déterminer le poids humide après élimination du surnageant. Les culots sont conservés à ≤-20°C.
Le reste de la culture est récolté en Nalgène de 50L et inactivé au phénol/éthanol (2% final V/V) et le polysaccharide est récupéré selon le protocole décrit dans l'exemple 2 ci-dessus.
L'ensemble des résultats obtenus est consigné dans le tableau 8 ci-dessous.

**Tableau 8 :**

| Milieux | Columbia + NaCl | Poutrel | BTS+ NaCl | M1 |
|---|---|---|---|---|
| DO finale | 21 | 16 | 17 | 16 |
| [CP5] extraction lysostaphine | nd | 6,6mg/l | nd | 130mg/l |
| [CP5] extraction autoclave | 0,2mg/l | 3,7mg/l | 3,5 mg/l | 40mg/l |
| Rendement spécifique (µg/DO) | 0,01 | 0,23 | 0,21 | 2,5 |

## Revendications

1. Milieu M0 adapté à la culture de *Staphylococcus aureus* comprenant :
• 3 à 50 g de peptone végétale,
• 0,5 à 20 g d'extrait de levure,
• 1 à 30 g de sucre,
• 1 à 200 mg d'un sel de fer sélectionné dans le groupe consistant en FeCl₃ et FeSO₄,
• 1 à 700 mg de sel de magnésium sélectionné dans le groupe consistant en MgCl₂ et Mg SO₄,
• 0,1 à 50 mg de CaCl₂,
• 0 à 50 mg de ZnCl₂,
• 2 à 50 g de NaCl, de préférence 6 g/l, et
dont le pH est compris dans une gamme de valeurs allant de 6, 2 à 7,5.

2. Un milieu M0 selon la revendication 1 comprenant :
(a) 3,87 ou 30 g de peptone de blé, et
(b) 5 g d'extrait de levure,
(c) 1 g de D-glucose H₂O,
(d) 120 mg de FeCl₃,6H₂O,
(e) 400 mg de MgCl₂,6H₂O,
(f) 10 mg de CaCl₂,2H₂O,
(g) 6 g de NaCl,
(h) 5 mg de ZnCl₂,
(i) 6.8 g de (NH₄)₂SO₄,
(j) 6 g de NH₄Cl et pH =6,5

## Claims

1. Culture medium M0 suitable for the culture of *Staphylococcus aureus* comprising :
• 3 to 50 g of plant peptone,
• 0,5 to 20 g of yeast extract,
• 1 to 30 g of sugar,
• 1 to 200 mg of an iron salt selected from the group consisting of FeCl₃ and FeSO₄,
• 1 to 700 mg of a magnesium salt selected from the group consisting of MgCl₂ and MgSO₄,
• 0,1 to 50 mg of CaCl₂,
• 0 to 50 mg of ZnCl₂,
• 2 to 50 g of NaCl, preferably 6 g/l and
The pH of which is within a range of values from 6,2 to 7,5.

2. Culture medium M0 according to claim 1, comprising:
(a) 3, 87 or 30 g of wheat peptone, and
(b) 5 g of yeast extract,
(c) 1 g of D-glucose, H₂O,
(d) 120 mg of FeCl₃, 6H₂O,
(e) 400 mg of MgCl₂, 6H₂O,
(f) 10 mg of CaCl₂, 2H₂O,
(g) 6 g of NaCl,
(h) 5 mg of ZnCl₂,
(i) 6,8 g of (NH₄)₂SO₄,
(j) 6 g of NH₄Cl and pH=6,5.

## Patentansprüche

1. Kulturmedium M0, an der *Staphylococcus aureus* kultur angepasst, umfassend:
• 3 bis 50 g pflanzliches peptone,
• 0,5 bis 20 g Hefeextrakt,
• 1 bis 30 g Zucher,
• 1 bis 200 mg eines Eisensalzes, ausgewält aus der gruppe, bestehend aus FeCl₃ und FeSO₄,
• 1 bis 700 mg eines Magnesiumsalzes, ausgewält aus der gruppe, bestehend aus MgCl₂ und MgSO₄,
• 0,1 bis 50 mg CaCl₂,
• 0 bis 50 mg ZnCl₂,
• 2 bis 50 g NaCl, vorzugsweise 6 g/l und
dessen pH -wert in einem bereich von 6,2 bis 7,5 liegt.

2. Kulturmedium M0 nach anspruch 1, umfassend:
(a) 3, 87 oder 30 g weizenpepton, und
(b) 5 g Hefeextrakt,
(c) 1 g D-glucose, H₂O,
(d) 120 mg FeCl₃, 6H₂O,
(e) 400 mg MgCl₂, 6H₂O,
(f) 10 mg CaCl₂, 2H₂O,
(g) 6 g NaCl,
(h) 5 mg ZnCl₂,
(i) 6,8 g (NH₄)₂SO₄,
(j) 6 g NH₄Cl und pH=6,5.
